# EUROPEAN PATENT APPLICATION

(11) **EP 4 397 609 A1**
(43) Date of publication of application: **10.07.2024**
(21) Application number: 23219971.1
(22) Date of filing: 22.12.2023
(51) Int. Cl.: B65H 59/40, B65H 63/00, B65H 69/00, G01N 3/08, G01N 33/36

(54) **YARN WINDING DEVICE AND AUTOMATIC WINDER**

(30) Priority: 04.01.2023 JP 2023000153
(71) Applicant: Murata Machinery, Ltd., Kyoto-shi, Kyoto 601-8326 (JP)
(72) Inventor: MIYAWAKI, Yasuhito, Fushimi-ku, Kyoto-shi, Kyoto, 612-8686 (JP); NAKAO, Ken, Fushimi-ku, Kyoto-shi, Kyoto, 612-8686 (JP); HASEGAWA, Kota, Fushimi-ku, Kyoto-shi, Kyoto, 612-8686 (JP)
(74) Representative: Stöckeler, Ferdinand

(57) **Abstract**

A first winder unit 3A includes a yarn supplying device 12 capable of supplying a yarn Y, a winding device 14 configured to wind the yarn Y supplied from the yarn supplying device 12 to form a package P, a holding device 21 configured to hold the yarn Y between the yarn supplying device 12 and the winding device 14, a yarn pulling device 25 configured to pull the yarn Y held by the holding device 21, and a tension measuring device 22 configured to measure a tension of the yarn Y pulled by the yarn pulling device 25, in which the tension measuring device 22 is connected to the holding device 21 or the yarn pulling device 25.

## Description

### TECHNICAL FIELD

The present invention relates to a yarn winding device and an automatic winder.

### BACKGROUND

As a conventional yarn winding device, for example, a yarn winding device described in Japanese Unexamined Patent Publication No. 2012-224431 is known. The yarn winding device described in Japanese Unexamined Patent Publication No. 2012-224431 includes a bobbin supporting section adapted to support a yarn supplying bobbin, a winding section adapted to wind a yarn of the yarn supplying bobbin supported by the bobbin supporting section as a package, and a strength measuring section adapted to measure strength of the yarn pulled out from the yarn supplying bobbin between the bobbin supporting section and the winding section.

### SUMMARY

In a conventional yarn winding device, a yarn is pulled by a winding section to measure a tension (breaking strength) of the yarn. Thus, in a configuration of pulling the yarn by the winding section, a pulling speed may not be stable, and as such the yarn may not be pulled by the pulling speed based on a predetermined tension test standard. Further, in the conventional yarn winding device, the tension of the yarn is measured by a tension sensor. Depending on the performance of the tension sensor used to form a package, a measuring range of the tension is narrow. Therefore, measurement of the strength of the yarn according to the tension test standard may not be realized.

One aspect of the present invention is to provide a yarn winding device and an automatic winder capable of realizing measurement of strength of a yarn conforming to a tension test standard.

A yarn winding device according to one aspect of the present invention includes: a yarn supplying section configured to supply a yarn; a winding section configured to wind the yarn supplied from the yarn supplying section to form a package; a holding section configured to hold the yarn between the yarn supplying section and the winding section; a pulling section configured to pull the yarn held by the holding section; and a measuring section configured to measure a tension of the yarn pulled by the pulling section, in which the measuring section is connected to the holding section or the pulling section.

A yarn winding device according to one aspect of the present invention includes: a holding section configured to hold a yarn; a pulling section configured to pull the yarn held by the holding section; and a measuring section configured to measure a tension of the yarn pulled by the pulling section. As described above, the yarn winding device includes the holding section, the pulling section, and the measuring section as a dedicated device for measuring the tension of the yarn. Thus, in the yarn winding device, the yarn can be pulled at a pulling speed based on a predetermined tension test standard. Furthermore, the measuring section can have a performance capable of measuring the strength of the yarn according to the tension test standard. Therefore, in the yarn winding device, the measurement of the strength of the yarn according to the tension test standard can be realized.

In one embodiment, the measuring section may be a load cell and connected to the holding section. In this configuration, the tension of the yarn can be accurately measured by using the load cell.

In one embodiment, the pulling section includes a winding portion having a locking member for hooking the yarn, the winding portion being configured to wind the yarn hooked to the locking member, and the measuring section may be connected to the pulling section and measures the rotational torque of the winding portion. In this configuration, the tension of the yarn can be measured with a simple configuration.

In one embodiment, the pulling section may include a driving section configured to rotationally drive the winding portion. In this configuration, the pulling speed of the yarn can be controlled by controlling the winding speed of the yarn in the winding section. Thus, the tension of the yarn can be appropriately measured.

In one embodiment, the driving section may be configured to rotate, after measurement of the yarn, the winding portion in a direction opposite to a direction in which the winding portion is rotated to wind the yarn, and the winding section may be configured to wind the yarn unwound from the winding portion when the winding portion is rotated in the direction opposite to the direction. When the elongation of the yarn is high, a large amount of yarns are wound around the winding portion. Therefore, after measuring the tension of the yarn, the winding portion is rotated in the direction opposite to the direction in which the yarn is wound to unwind the yarn. At this time, the winding section winds the yarn fed out from the winding portion around the package. Thus, in the case of a yarn having high elongation, the yarn can be suppressed from remaining in the winding portion (state of being wound) even when a large amount of yarns are wound around the winding portion.

In one embodiment, the pulling section may be arranged so as to be movable in a forward-and-rearward direction with respect to a yarn path of the yarn. In this configuration, the yarn can be pulled at a position where the yarn deviates from the yarn path by moving the pulling section. Therefore, the yarn can be avoided from being caught by guidance and the like at the time of measuring the tension. Accordingly, deterioration in the measurement accuracy of the tension of the yarn can be suppressed.

In one embodiment, the yarn winding device may include a display section configured to display information on the measurement of the yarn in the measuring section. In this configuration, the information on the measurement of the yarn can be confirmed in the display section. Therefore, for example, it is not necessary to move to a device installed at a distant position to confirm measurement data. Therefore, the workload of an operator can be reduced.

In one embodiment, the yarn winding device may include an operating section configured to receive an input related to measurement of the tension of the yarn, and a control section configured to execute an operation related to the measurement of the yarn based on the input received in the operating section. In this configuration, in the yarn winding device, various operations related to the tension measurement of the yarn to be measured, such as selecting a measurement item of the yarn and erasing the measurement data, can be executed by operating the operating section.

In one embodiment, the yarn winding device may include: a yarn joining section configured to perform yarn joining of the yarn from the yarn supplying section and the yarn from the winding section between the holding section and the pulling section; a first catching section configured to catch the yarn from the winding section and to guide the yarn to the yarn joining section; and a second catching section configured to catch the yarn from the yarn supplying section and to guide the yarn to the yarn joining section, in which the second catching section may catch, after the measurement of the yarn, the yarn from the yarn supplying section and move the yarn above the holding section. In this configuration, the yarn can be suppressed from remaining in the holding section after the tension measurement of the yarn.

In one embodiment, the yarn winding device may include a setting section configured to change, based on a measurement result of the tension of the yarn, a setting related to a yarn joining operation in the yarn joining section. In this configuration, the setting (various parameters) related to the yarn joining operation of the yarn joining section can be automatically changed (set). Therefore, the workload of the operator can be reduced.

In one embodiment, the yarn winding device may include: a yarn monitoring section configured to monitor a state of the yarn travelling through the yarn path of the yarn between the holding section and the pulling section, and to calculate a length of the yarn based on monitored information; and a calculating section configured to calculate, when the yarn held by the holding section is pulled by the pulling section, an elongation of the yarn based on the length of the yarn calculated by the yarn monitoring section. In this configuration, the elongation of the yarn can be calculated in the measurement of the tension of the yarn.

In one embodiment, the yarn winding device may include a calculating section configured to calculate, when the yarn held by the holding section is pulled by the pulling section, an elongation of the yarn based on a time between a start and an end of pulling of the yarn by the pulling section and a pulling speed of the yarn. In this configuration, the elongation of the yarn can be calculated in the measurement of the tension of the yarn.

An automatic winder according to one aspect of the present invention is an automatic winder including: a plurality of yarn winding units configured to wind a yarn obtained by unwinding the yarn from a yarn supplying bobbin to form a package; and a central control section configured to control the plurality of yarn winding units, in which at least one of the plurality of yarn winding units is any one of the yarn winding devices described above.

The automatic winder according to one aspect of the present invention includes a plurality of yarn winding units, and at least one of the plurality of yarn winding units is any one of the yarn winding devices described above. Therefore, in the automatic winder, the measurement of the strength of the yarn according to the tension test standard can be realized.

In one embodiment, the central control section may be arranged at an end portion in a juxtaposition direction in which the plurality of yarn winding units are arranged side by side, and the yarn winding device may be arranged at a position closest to the central control section in the juxtaposition direction. In this configuration, since the yarn winding device is arranged near the central control section, the tension test can be efficiently carried out when the measurement result in the yarn winding device is managed in the central control section or the like.

According to one aspect of the present invention, measurement of strength of a yarn conforming to a tension test standard can be realized.

### BRIEF DESCRIPTION OF THE DRAWINGS

FIG. 1 is a front view illustrating an automatic winder according to an embodiment;
FIG. 2 is a side view schematically illustrating a first winder unit of FIG. 1;
FIG. 3 is a side view schematically illustrating a second winder unit of FIG. 1;
FIG. 4 is a perspective view illustrating the first winder unit;
FIG. 5 is a front view illustrating the first winder unit;
FIG. 6 is a side view illustrating the first winder unit;
FIG. 7 is a view illustrating a display device;
FIG. 8 is a perspective view illustrating a holding device and a tension measuring device;
FIG. 9 is a front view illustrating the holding device and the tension measuring device;
FIG. 10 is a side view illustrating the holding device and the tension measuring device;
FIG. 11 is a perspective view illustrating a yarn pulling device at a standby position;
FIG. 12 is a front view illustrating the yarn pulling device at the standby position;
FIG. 13 is a side view illustrating the yarn pulling device at the standby position;
FIG. 14 is a perspective view illustrating the yarn pulling device at a pulling position;
FIG. 15 is a front view illustrating the yarn pulling device at the pulling position;
FIG. 16 is a side view illustrating the yarn pulling device at the pulling position;
FIG. 17 is a perspective view illustrating a part of a first winder unit according to another embodiment; and
FIG. 18 is a perspective view illustrating a part of the first winder unit according to another embodiment.

### DETAILED DESCRIPTION

Hereinafter, preferred embodiments of the present invention will be described in detail with reference to the accompanying drawings. In the description of the drawings, the same or corresponding elements are denoted by the same reference numerals, and redundant description is omitted.

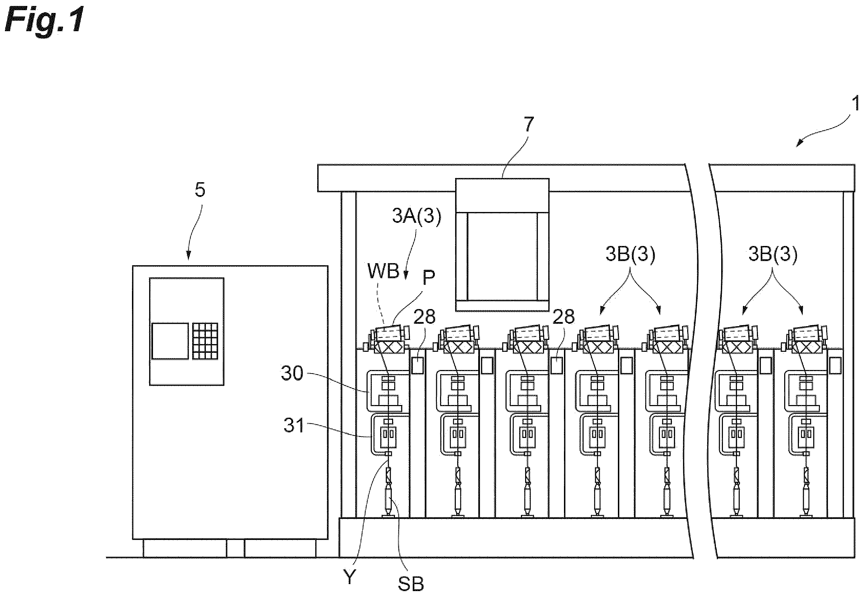

As illustrated in FIG. 1, an automatic winder (yarn winding machine) 1 includes a plurality of winder units (yarn winding units) 3 arranged in line, a machine control device 5, and a doffing device 7. The machine control device (central control section) 5 can communicate with each of the plurality of winder units 3. An operator of the automatic winder 1 can collectively manage the plurality of winder units 3 by appropriately operating the machine control device 5. When a package P is fully wound (state in which a predetermined amount of yarn is wound) in a second winder unit 3B (described later), the doffing device 7 travels to a position of the second winder unit 3B, detaches the fully wound package, and sets an empty winding bobbin WB.

The plurality of winder units 3 include a first winder unit (a yarn winding device) 3A and the second winder unit 3B. In the present embodiment, the plurality of winder units 3 includes one first winder unit 3A and a plurality of second winder units 3B. The first winder unit 3A is arranged, for example, at an end (a position closest to the machine control device 5) on the machine control device 5 side in the arrangement direction of the plurality of winder units 3. The automatic winder 1 may include a plurality of first winder units 3A.

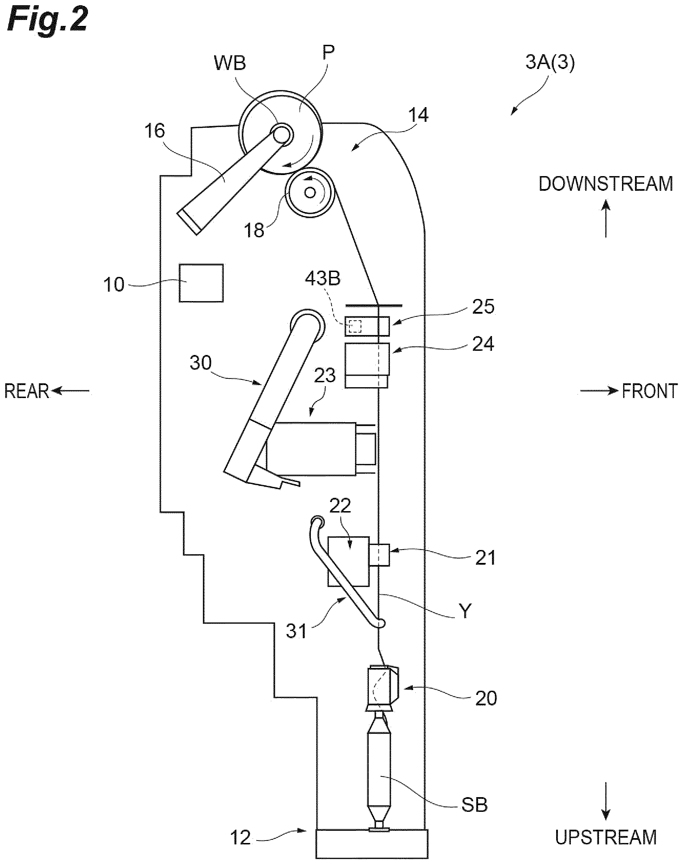

As illustrated in FIG. 2, the first winder unit 3A measures the tension (breaking strength) of a yarn Y. The first winder unit 3A includes a section controller (a control section, a setting section, and a calculating section) 10, a yarn supplying device (a yarn supplying section) 12, and a winding device (a winding section) 14. The section controller 10 includes, for example, a central processing section (CPU) and a read only memory (ROM) . The ROM stores a program for controlling each configuration of the winder unit 3. The CPU executes the program stored in the ROM. The section controller 10 controls the operation of each section in the first winder unit 3A.

The yarn supplying device 12 supports a yarn supplying bobbin SB placed on a conveyance tray (not illustrated) at a predetermined position. The yarn supplying device 12 unwinds the yarn Y from the yarn supplying bobbin SB, and pulls out the yarn Y from the yarn supplying bobbin SB. The yarn supplying device 12 supplies the yarn Y. The yarn supplying device 12 is not limited to the conveyance tray type device, and may be, for example, a magazine type device, or may be, for example, a device adapted to supply the yarn Y from a cone type yarn supplying package.

The winding device 14 includes a cradle 16 and a winding drum 18. The cradle 16 sandwiches the winding bobbin WB to rotatably support the winding bobbin WB (or the package P). The winding drum 18 traverses the yarn Y on the surface of the package P and rotates the package P. The winding drum 18 is rotationally driven by a drum driving motor (not illustrated). The package P is driven and rotated by rotationally driving the winding drum 18 in a state in which the outer periphery of the package P is in contact with the winding drum 18. A spiral traverse groove is formed on the outer peripheral surface of the winding drum 18. The yarn Y unwound from the yarn supplying bobbin SB is wound around the surface of the package P while being traversed with a constant width by the traverse groove. Thus, the package P having a constant winding width can be formed.

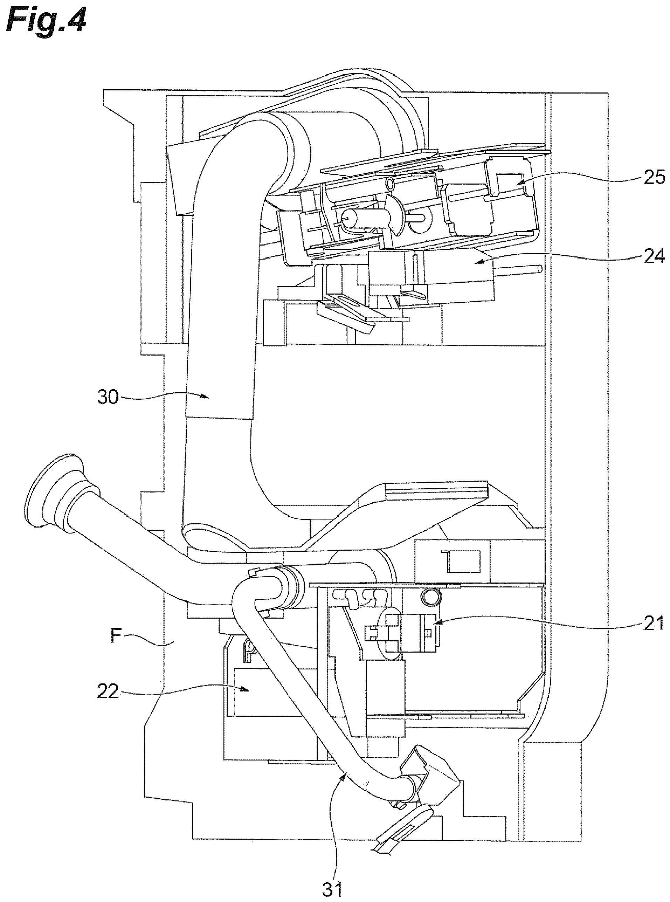

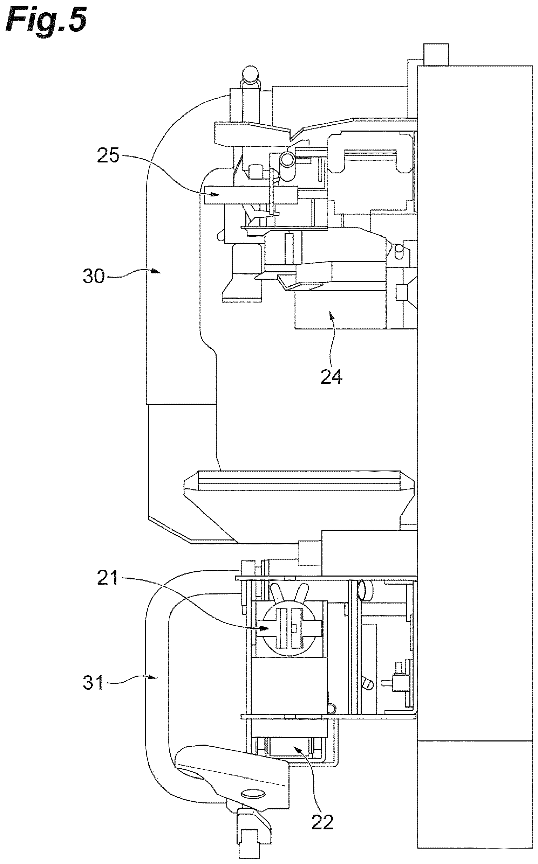

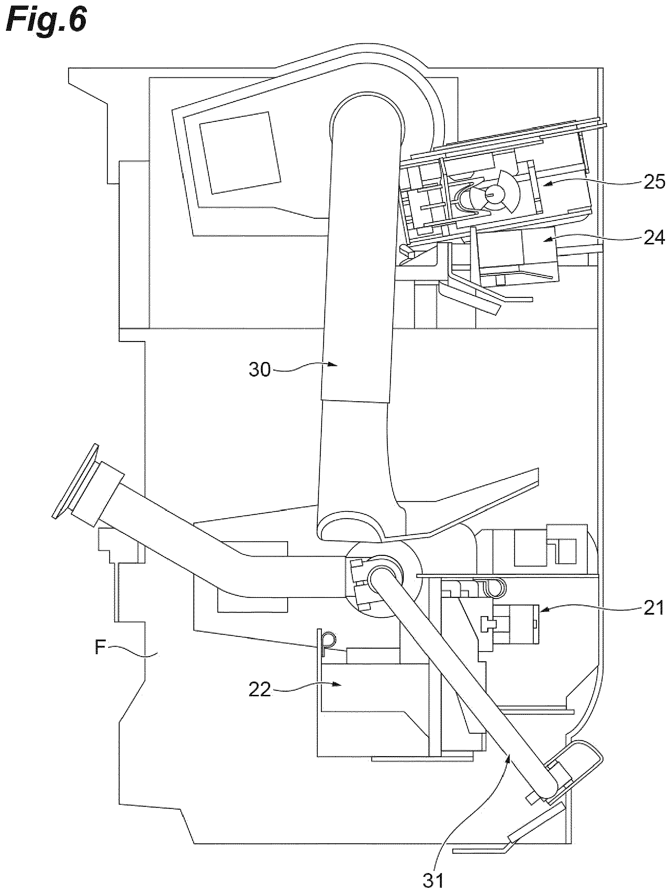

As illustrated in FIGS. 2, 4, 5, and 6, the first winder unit 3A includes an unwinding assisting device 20, a holding device (a holding section) 21, a tension measuring device (measuring section) 22, a yarn joining device (a yarn joining section) 23, a yarn monitoring device (a yarn monitoring section) 24, and a yarn pulling device (a pulling section) 25 in this order from the yarn supplying device 12 along a yarn path formed between the yarn supplying device 12 and the winding device 14. The first winder unit 3A includes a display device 26. A first catching and guiding device (a first catching section) 30 and a second catching and guiding device (a second catching section) 31 are arranged in proximity to the yarn joining device 23. In FIGS. 4 to 6, illustration of the unwinding assisting device 20, the yarn joining device 23, and the display device 26 is omitted.

The unwinding assisting device 20 prevents the yarn Y unwound from the yarn supplying bobbin SB from being excessively swung by a centrifugal force, and appropriately unwinds the yarn Y from the yarn supplying bobbin SB.

The holding device 21 holds the yarn Y. In the present embodiment, the holding device 21 holds the yarn Y by gripping the yarn Y. The tension measuring device 22 measures the tension of the yarn Y. The tension measuring device 22 measures the tension of the yarn Y pulled by the yarn pulling device 25. Detailed configurations of the holding device 21 and the tension measuring device 22 will be described later.

When the yarn Y is disconnected for some reason between the yarn supplying device 12 and the winding device 14, the yarn joining device 23 joins the yarn Y from the yarn supplying device 12 and the yarn Y from the winding device 14.

The yarn monitoring device 24 monitors the state of the yarn Y travelling through the yarn path, and detects the presence or absence of a yarn defect based on monitored information. The yarn defect is, for example, at least one of thickness abnormality of the yarn Y, a foreign substance contained in the yarn Y, yarn breakage, and the like. The yarn monitoring device 24 may detect the speed of the yarn Y based on the monitored information. The yarn monitoring device 24 may calculate the length of the yarn Y passing through the yarn monitoring device 24 based on the detected speed of the yarn Y.

The yarn pulling device 25 pulls the yarn Y. The yarn pulling device 25 is arranged between the winding device 14 and the yarn monitoring device 24. The yarn pulling device 25 pulls the yarn Y held by the holding device 21. A detailed configuration of the yarn pulling device 25 will be described later.

The display device 26 displays information relating to the tension measurement of the yarn Y in the tension measuring device 22. The display device 26 is arranged at a position operable by an operator in the first winder unit 3A. The display device 26 is arranged, for example, at a front portion of the first winder unit 3A.

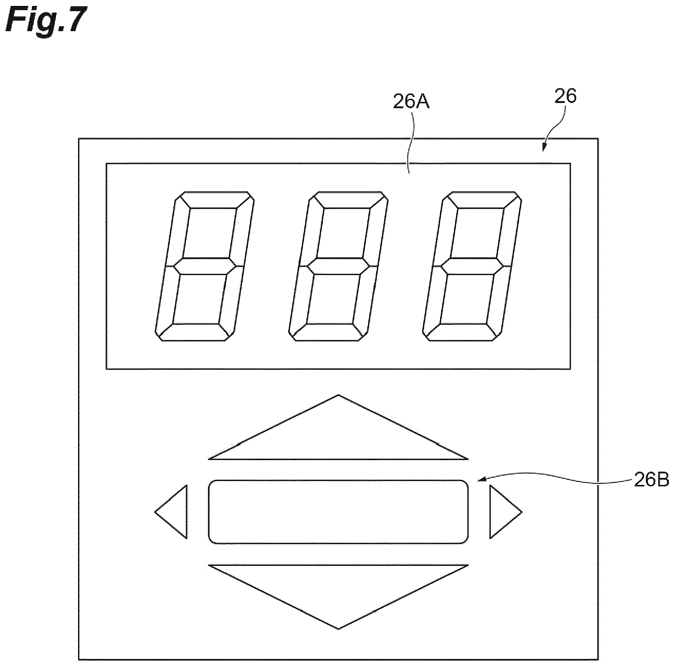

As illustrated in FIG. 7, the display device 26 includes a display section 26A and an operating section 26B. In the present embodiment, the display section 26A is a 7-segment display that displays characters by a plurality of segments. The characters displayed on the display section 26A are, for example, numbers (Arabic numerals), alphabets (Latin characters), and the like. The operating section 26B is a portion operated by the operator, and receives an input from the operator. The operating section 26B includes a selection button for selecting a display item or the like on the display section 26A, a determination button, and the like. The display content on the display section 26A can be changed by the operation of the operating section 26B by the operator.

The number of times of measurement of the tension of the yarn Y in the first winder unit 3A and a measurement item of the yarn Y are displayed on the display section 26A. The measurement item includes the tension (joint strength) of a yarn joining portion joined by the yarn joining device 23 and the tension of the yarn Y itself. The number of times of measurements is indicated by a number. As the measurement item, a letter indicating the measured tension of the yarn Y itself is displayed. For example, when a measured value is "100cN", "100" is displayed on the display section 26A. The display switching between the number of times of measurement and the measurement item may be automatically performed at any timing or may be performed by the operation of the operating section 26B.

Measurement data by the tension measuring device 22 is displayed on the display section 26A. The display section 26A displays an average value of the tension of the yarn joining portion of the yarn Y, an average value of the tension of the yarn Y itself, a minimum value of the tension of the yarn joining portion of the yarn Y, a minimum value of the tension of the yarn Y itself, CV% (a fluctuation rate) of the tension of the yarn joining portion of the yarn Y, CV% (a fluctuation rate) of the tension of the yarn Y itself, and a retention rate (an average value of the tension of the yarn joining portion of yarn Y/an average value of the tension of the yarn Y itself × 100). These values are calculated by the section controller 10 based on the stored measurement data. The operator can display the various pieces of data on the display section 26A by operating the operating section 26B.

In the display device 26, the measurement data can be erased. By operating the operating section 26B, the operator can display the measurement data to be erased on the display section 26A and erase the measurement data. In addition, the measurement of the measurement item corresponding to the erased measurement data can be performed. Specifically, for example, after the measurement data is erased, the measurement of the measurement item of the measurement data can be performed manually (by operating the operating section 26B) or automatically. The measurement data and the like can also be displayed in the machine control device 5.

As illustrated in FIGS. 2, 4, 5, and 6, the first catching and guiding device 30 is swingable from a standby position on the yarn supplying device 12 side to a catching position on the winding device 14 side. The first catching and guiding device 30 catches the yarn Y at the catching position and guides the yarn Y to the yarn joining device 23. The second catching and guiding device 31 is swingable from the standby position on the yarn supplying device 12 side to the catching position on the winding device 14 side. The second catching and guiding device 31 catches the yarn Y at the catching position and guides the yarn Y to the yarn joining device 23.

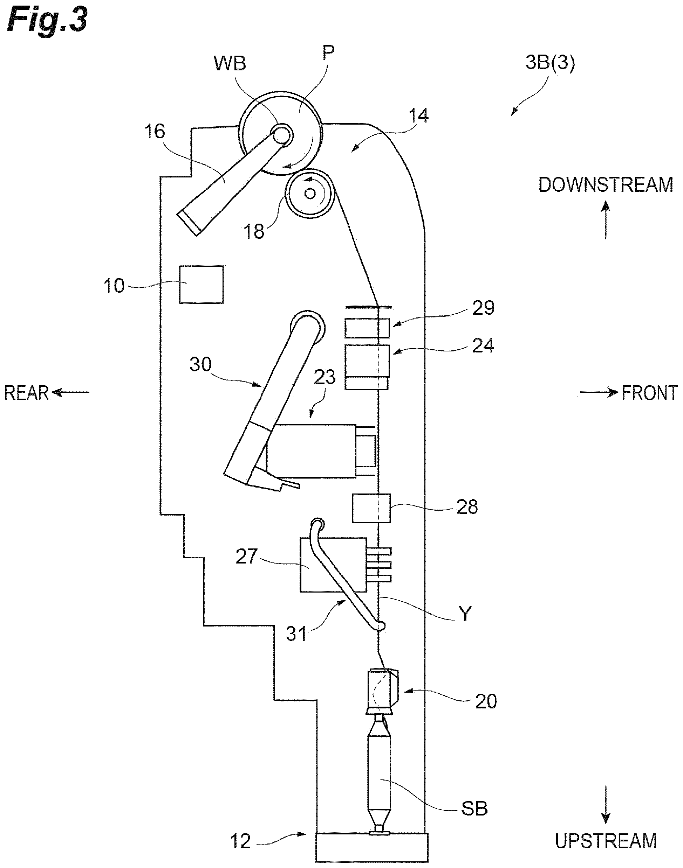

As illustrated in FIG. 3, each second winder unit 3B forms the package P by unwinding the yarn Y from the yarn supplying bobbin SB and winding the yarn Y around the winding bobbin WB while traversing the yarn Y.

The second winder unit 3B includes a section controller 10, a yarn supplying device 12, and a winding device 14. The section controller 10 controls the operation of each section in the second winder unit 3B.

Each second winder unit 3B includes an unwinding assisting device 20, a tension applying device 27, a tension detecting device 28, a yarn joining device 23, a yarn monitoring device 24, and a wax applying device 29 arranged in this order from the yarn supplying device 12 along the yarn path formed between the yarn supplying device 12 and the winding device 14. The second winder unit 3B includes the display device 26. A first catching and guiding device 30 and a second catching and guiding device 31 are arranged in proximity to the yarn joining device 23.

The tension applying device 27 applies a predetermined tension on the travelling yarn Y. In the present embodiment, the tension applying device 27 is a gate-type device in which movable comb teeth are arranged with respect to fixed comb teeth. The tension detecting device 28 detects the tension of the travelling yarn Y between the yarn supplying device 12 and the winding device 14.

The wax applying device 29 is arranged between the winding device 14 and the yarn monitoring device 24. The wax applying device 29 applies wax to the yarn Y travelling from the yarn monitoring device 24 toward the winding device 14.

The first winder unit 3A can be configured as the second winder unit 3B. When configuring the first winder unit 3A as the second winder unit 3B, the tension applying device 27, the tension detecting device 28, and the wax applying device 29 are mounted instead of the holding device 21 and the yarn pulling device 25. Specifically, the wax applying device 29 is mounted at a mounting position of the yarn pulling device 25.

The second winder unit 3B can be configured as the first winder unit 3A. When configuring the second winder unit 3B as the first winder unit 3A, the holding device 21 and the yarn pulling device 25 are mounted instead of the tension applying device 27, the tension detecting device 28, and the wax applying device 29.

Next, configurations of the holding device 21, the tension measuring device 22, and the yarn pulling device 25 will be described in detail.

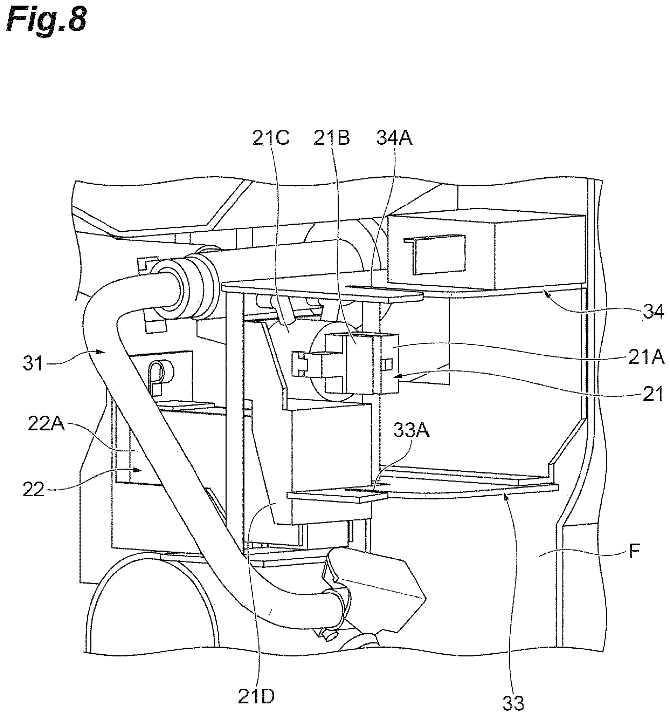

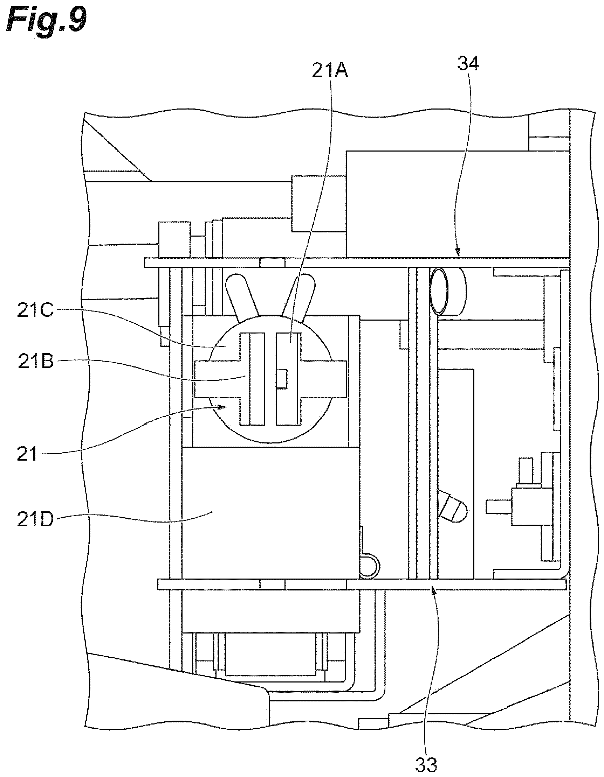

As illustrated in FIGS. 8, 9, and 10, the holding device 21 includes a first gripping portion 21A, a second gripping portion 21B, and a driving section 21C. The first gripping portion 21A and the second gripping portion 21B are arranged to face each other at positions sandwiching the yarn path of the yarn Y from the left and right. The first gripping portion 21A and the second gripping portion 21B are formed of, for example, metal such as iron. In the present embodiment, a slip suppressing member such as urethane that suppresses the slip of the yarn Y is arranged at a portion in contact with the yarn Y in the first gripping portion 21A. In the second gripping portion 21B, a roughening process of roughening the surface of metal by shot blasting is performed at a portion in contact with the yarn Y for the purpose of suppressing the slip of the yarn Y.

The holding device 21 is disposed between a first guidance 33 and a second guidance 34. The first guidance 33 is arranged on the yarn supplying device 12 side. The second guidance 34 is arranged on the winding device 14 side. The first guidance 33 is provided with a guidance portion 33A through which the yarn Y travels. The second guidance 34 is provided with a guidance portion 34A through which the yarn Y travels. The guidance portion 33A and the guidance portion 34A are arranged on a straight line along the travelling direction of the yarn Y. A member (for example, a brush or the like) adapted to prevent the yarn Y from falling may be arranged on the guidance portion 33A of the first guidance 33. Thus, when yarn breakage occurs, the yarn Y can be suppressed from falling to the yarn supplying bobbin SB.

The driving section 21C drives the first gripping portion 21A and the second gripping portion 21B. For example, the driving section 21C drives the first gripping portion 21A and the second gripping portion 21B with compressed air. By the driving of the driving section 21C, the first gripping portion 21A and the second gripping portion 21B move to a gripping position (a position at which the first gripping portion 21A and the second gripping portion 21B approach each other) of gripping (holding) the yarn Y and a standby position (a position at which the first gripping portion 21A and the second gripping portion 21B separate from each other) of not gripping the yarn Y. The operation of the driving section 21C is controlled by the section controller 10.

The holding device 21 holds the yarn Y in the tension measurement of the yarn Y. Further, the holding device 21 is operated to hold the yarn Y when yarn breakage is detected in the yarn monitoring device 24. Thus, when yarn breakage occurs, the yarn Y can be suppressed from falling to the yarn supplying bobbin SB.

The tension measuring device 22 is connected to the holding device 21. The holding device 21 and the tension measuring device 22 are connected to each other via a mounting member 21D. The holding device 21 is supported by the tension measuring device 22 via the mounting member 21D. The tension measuring device 22 measures a force applied to the holding device 21 when the yarn Y is pulled by the yarn pulling device 25 in a state in which the yarn Y is held by the holding device 21.

The tension measuring device 22 is a load cell. The measurement range of the tension measuring device 22 is, for example, 0 to 30N. The tension measuring device 22 includes a housing 22A. The housing 22A is fixed to a frame F. A strain element (not illustrated) is accommodated in the housing 22A. A plurality of strain gauges (not illustrated) are attached to the strain element. The plurality of strain gauges are connected to a Wheatstone bridge circuit (not illustrated). The tension measuring device 22 includes an A/D converter (not illustrated) in addition to the strain element. The tension measuring device 22 extracts, from the Wheatstone bridge circuit, an electric signal corresponding to a load transmitted from the strain element. The electric signal is obtained when the yarn Y is being pulled by the yarn pulling device 25. The electric signal is converted into a digital signal by the A/D converter. The tension measuring device 22 outputs the digital signal as the measurement data to the section controller 10.

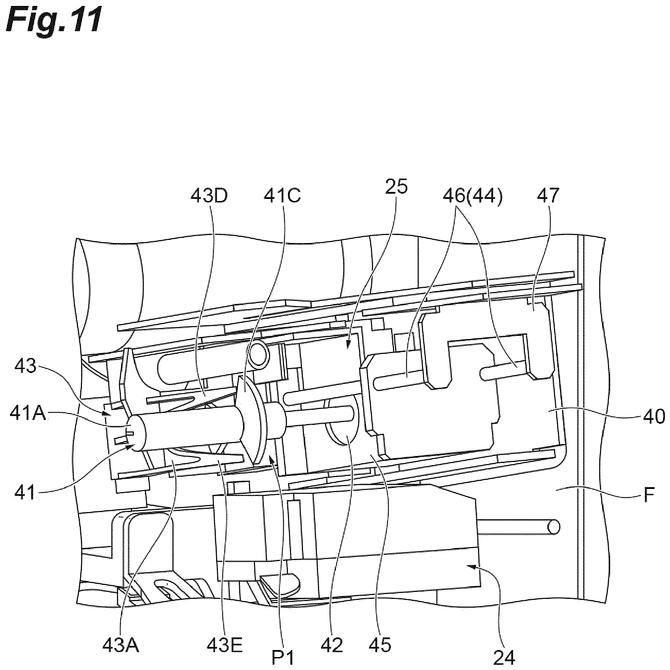

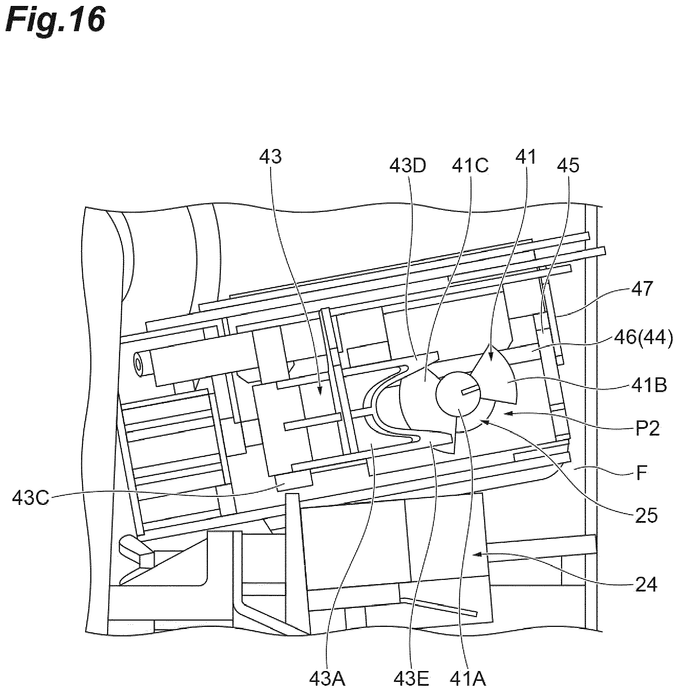

As illustrated in FIGS. 11 to 16, the yarn pulling device 25 includes a housing 40, a winding portion 41, a driving section 42, a detecting section 43, and a moving mechanism 44.

The housing 40 is fixed to the frame F. The winding portion 41, the driving section 42, the detecting section 43, and the moving mechanism 44 are mounted in the housing 40.

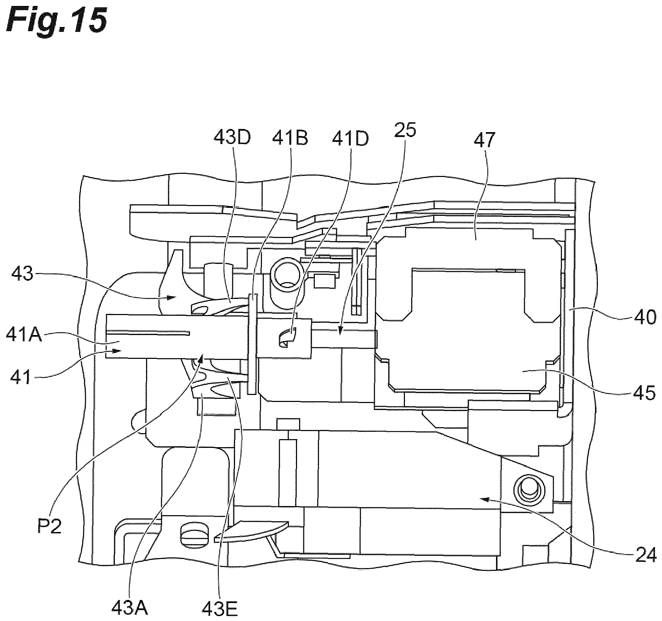

The winding portion 41 winds and pulls the yarn Y. The winding portion 41 includes a shaft portion 41A, a first cam portion 41B, and a second cam portion 41C. The shaft portion 41A is connected to the driving section 42. The shaft portion 41A is rotated by driving of the driving section 42. The shaft portion 41A is provided with a locking member 41D (refer to FIG. 15). The locking member 41D has a hook shape. The locking member 41D hooks the yarn Y by the rotation of the shaft portion 41A. In the winding portion 41, the yarn Y is hooked by the locking member 41D, and the yarn Y is wound around the shaft portion 41A by the rotation of the shaft portion 41A.

The first cam portion 41B and the second cam portion 41C are provided on the shaft portion 41A. Each of the first cam portion 41B and the second cam portion 41C has a fan shape. The first cam portion 41B and the second cam portion 41C are provided so as to protrude in the radial direction from the shaft portion 41A when viewed from the extending direction of the shaft portion 41A. The first cam portion 41B is smaller than the second cam portion 41C. The first cam portion 41B and the second cam portion 41C are disposed at symmetrical positions with respect to the shaft portion 41A when viewed from the extending direction of the shaft portion 41A. The first cam portion 41B is disposed at a position overlapping the locking member 41D when viewed from the extending direction of the shaft portion 41A.

The driving section 42 rotationally drives the winding portion 41. In the present embodiment, the driving section 42 is a stepping motor. An output shaft of the driving section 42 is connected to the shaft portion 41A. The driving section 42 is held by a holding section 45. The operation of the driving section 42 is controlled by the section controller 10.

The detecting section 43 detects the rotation position of the winding portion 41. The detecting section 43 includes a lever portion 43A and a sensor 43B (refer to FIG. 2). The lever portion 43A is provided to be swingable around a shaft portion 43C. A distal end portion of the lever portion 43A (an end portion on a side opposite to a proximal end portion pivotally supported by the shaft portion 43C) substantially has a U shape. The distal end portion of the lever portion 43A is configured by a first end portion 43D and a second end portion 43E. The first end portion 43D and the second end portion 43E have a curved shape.

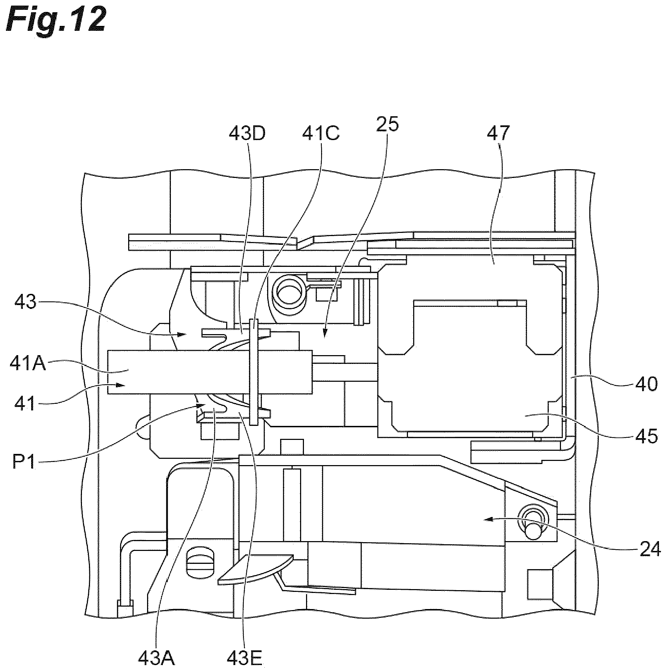

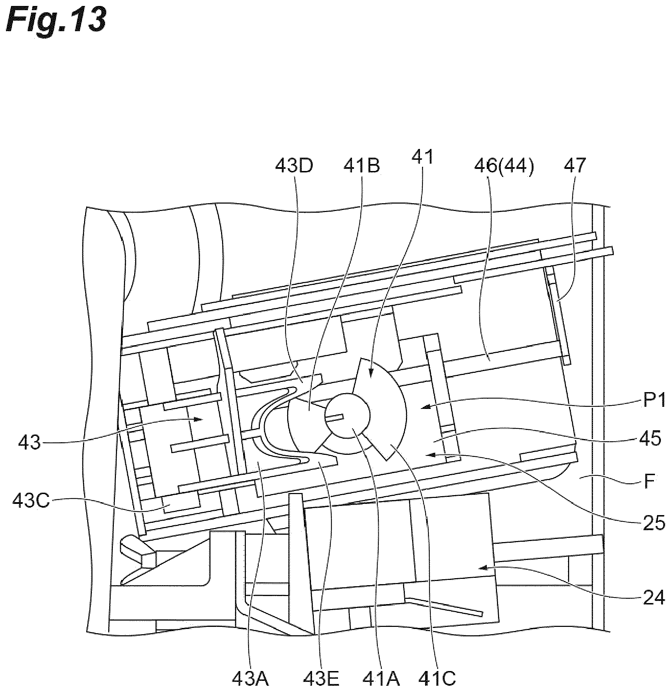

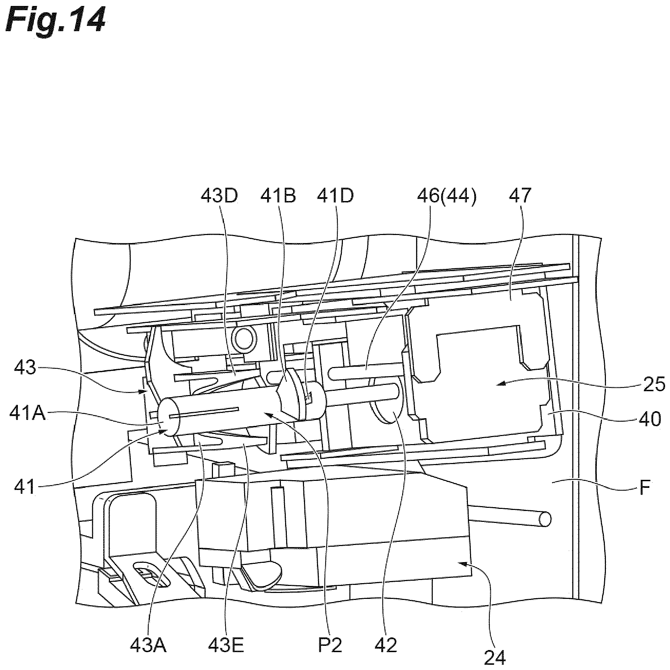

The lever portion 43A swings between a closed position (refer to FIG. 12) and an open position (refer to FIG. 15). The lever portion 43A swings according to the movement of the first cam portion 41B and the second cam portion 41C of the winding portion 41. Specifically, as illustrated in FIGS. 11 and 13, the first end portion 43D and the second end portion 43E of the lever portion 43A are located between the first cam portion 41B and the second cam portion 41C, and as illustrated in FIG. 12, when the first cam portion 41B is located between the first end portion 43D and the second end portion 43E when viewed from the forward-and-rearward direction, the lever portion 43A is located at the closed position. When the lever portion 43A is at the closed position, the winding portion 41 is at the initial position. At the initial position of the winding portion 41, the locking member 41D does not face the yarn path side of the yarn Y. When the first end portion 43D and the second end portion 43E of the lever portion 43A are in contact with the first cam portion 41B and/or the second cam portion 41C, the lever portion 43A is located at the open position. In the example illustrated in FIGS. 14 to 16, the lever portion 43A is in contact with the second cam portion 41C.

The sensor 43B detects the lever portion 43A. The sensor 43B detects (turns on) the lever portion 43A when the lever portion 43A is at the closed position (refer to FIG. 12). The sensor 43B outputs a detection result to the section controller 10.

The moving mechanism 44 is a mechanism for moving the winding portion 41 (the holding section 45) in the forward-and-rearward direction with respect to the yarn path of the yarn Y. That is, the winding portion 41 is arranged to be movable in the forward-and-rearward direction with respect to the yarn path of the yarn Y. The moving mechanism 44 moves the winding portion 41 to a standby position (a first position) P1 (refer to FIGS. 11 to 13) and a measuring position (a second position) P2 (refer to FIGS. 14 to 16). The standby position P1 is a position at which the winding portion 41 is located on the rear side with respect to the yarn path of the yarn Y. The measuring position P2 is a position at which the winding portion 41 is located on the front side with respect to the yarn path of the yarn Y. At the measuring position P2, the winding portion 41 pushes out the yarn Y forwards, and the yarn Y is detached from the yarn path.

The moving mechanism 44 includes two (a plurality of) slide rails 46 and a driving section (not illustrated). The two slide rails 46 are fixed to the housing 40 by a fitting 47. As illustrated in FIG. 13, the two slide rails 46 are disposed so as to be inclined upwards toward the front side. The driving section moves the holding section 45 along the two slide rails 46. The driving section is, for example, an air cylinder.

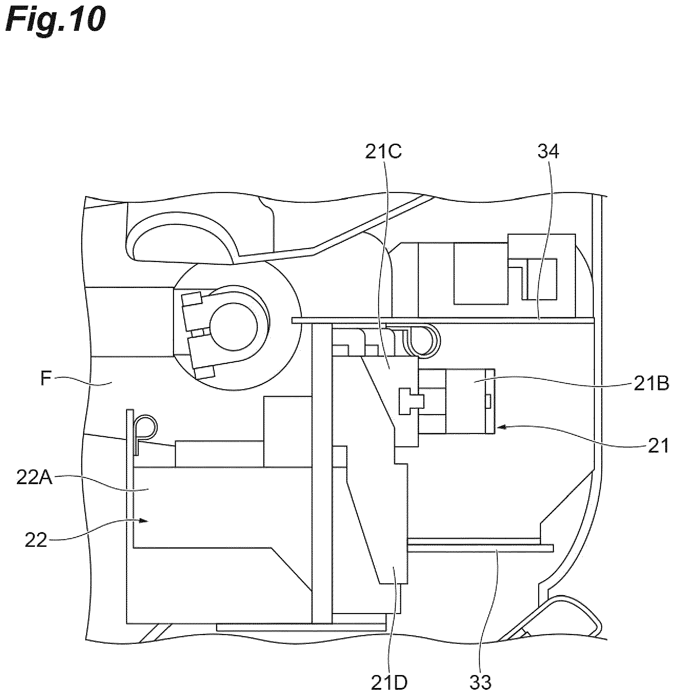

As illustrated in FIGS. 10 to 13, in the yarn pulling device 25, the winding portion 41 is located at the standby position P1 when the tension of the yarn Y is not measured. At this time, the locking member 41D of the winding portion 41 is located on the rear side. The lever portion 43A is located at the closed position. That is, the locking member 41D does not face the yarn path side of the yarn Y. Thus, the yarn Y can be avoided from being caught at the locking member 41D.

As illustrated in FIGS. 13 to 16, in the yarn pulling device 25, when the tension measurement of the yarn Y is started, the winding portion 41 moves to the measuring position P2. At this time, the locking member 41D of the winding portion 41 is located on the front side. That is, the locking member 41D faces the yarn path side of the yarn Y. Thus, the yarn Y can be hooked to the locking member 41D by rotating the winding portion 41. The yarn pulling device 25 rotates the winding portion 41 to pull the yarn Y until the yarn Y is broken (cut) . When the yarn Y is broken, the yarn pulling device 25 stops the rotation of the winding portion 41. Thereafter, in the yarn pulling device 25, the winding portion 41 is moved to the standby position P1.

Next, an operation related to the tension measurement of the yarn Y in the first winder unit 3A will be described. The first winder unit 3A executes measurement of the tension of the yarn Y by the operation of the operator via the machine control device 5 and the section controller 10. The operator selects the measurement item by operating the operating section 26B of the display device 26. In the present embodiment, the measurement item includes two items of measurement of the tension of the yarn joining portion joined by the yarn joining device 23 and measurement of the tension of the yarn Y itself. The measurement of the tension of the yarn joining portion means measuring the tension until the yarn joining portion is broken. The measurement of the tension of the yarn Y itself means measuring the tension until the yarn Y itself is broken. The measurement of the tension of the yarn joining portion of the yarn Y may be carried out after changing (adjusting) the setting of the yarn joining device 23 or the like, or may be carried out periodically. The measurement of the tension of the yarn Y may be carried out after changing a lot or the like, or may be carried out periodically.

### [Measurement of Tension Until Yarn Joining Portion Is Broken]

First, the measurement of the tension of the yarn joining portion joined by the yarn joining device 23 will be described. In the first winder unit 3A, when the measurement of the tension of the yarn joining portion is selected in the operating section 26B and the measurement is started, the yarn joining device 23 executes a yarn joining operation. At this time, in the yarn pulling device 25, the shaft portion 41A of the winding portion 41 is rotated at the standby position P1, and the shaft portion 41A is located at the initial position based on the detection result of the sensor 43B of the detecting section 43.

In the first winder unit 3A, when the yarn joining operation is completed, the yarn Y is caught and pulled by the second catching and guiding device 31 to eliminate the loosening of the yarn Y, and the holding device 21 holds the yarn Y. Further, in the yarn pulling device 25, the shaft portion 41A of the winding portion 41 is rotated to position the locking member 41D on the yarn path side. Subsequently, in the yarn pulling device 25, the winding portion 41 is moved from the standby position P1 to the measuring position P2 by the moving mechanism 44, and for example, the winding portion 41 is rotated counterclockwise. Thus, the yarn pulling device 25 hooks the yarn Y deviated from the yarn path using the locking member 41D, and pulls the yarn Y by winding the yarn Y around the shaft portion 41A. Since the yarn joining device 23 is arranged between the holding device 21 and the yarn pulling device 25 in the yarn path of the yarn Y, the yarn joining portion joined by the yarn joining device 23 is pulled. The tension measuring device 22 measures the tension until the yarn Y pulled by the yarn pulling device 25 is broken. The tension measuring device 22 outputs a measurement result until the yarn Y is broken to the section controller 10. The section controller 10 displays the measured tension of the yarn Y on the display section 26A of the display device 26.

### [Measurement of Tension Until Yarn Itself Is Broken]

Next, the measurement of the tension of the yarn Y itself will be described. In the first winder unit 3A, the measurement of the tension of a main body of the yarn Y is selected in the operating section 26B, and the measurement is started. When the yarn Y supplied from the yarn supplying device 12 is in a state of being wound by the winding device 14 (state in which the yarn Y is connected between the yarn supplying device 12 and the winding device 14), the measurement of the tension of the yarn Y is immediately started. When the yarn Y supplied from the yarn supplying device 12 is not in the state of being wound by the winding device 14, the yarn joining device 23 executes a yarn joining operation. When the yarn joining device 23 executes the yarn joining operation, the yarn Y is wound by a predetermined amount by the winding device 14. Thus, since the yarn joining portion does not exist between the holding device 21 and the yarn pulling device 25, the tension of the yarn Y can be measured. In this state, the tension until the yarn Y is broken is measured in the same manner as the measurement of the tension of the yarn joining portion.

In the first winder unit 3A, in the yarn pulling device 25, after the yarn Y is broken, the winding portion 41 is rotated in a direction opposite to the direction in which the yarn Y is wound, thereby feeding out the yarn Y (unwinding the yarn Y). At this time, in the winding device 14, the winding drum 18 is rotated to wind the yarn Y fed out from the winding portion 41 around the package P. Thus, the yarn Y can be suppressed from remaining on the winding portion 41 (in the state of being wound). In particular, when the elongation of the yarn Y is high, a large amount of the yarn Y is wound around the winding portion 41 until the yarn Y is broken as compared with the yarn Y having a general elongation. Therefore, in the case of the yarn Y having a high elongation, the above-described operation after the yarn Y is broken is effective for suppressing the yarn Y from remaining on the winding portion 41 around which the large amount of yarns Y are wound.

In the first winder unit 3A, processing of removing the yarn Y from the holding device 21 is executed after measuring the tension of the yarn Y (after the yarn Y is broken). Specifically, in the first winder unit 3A, the yarn Y from the yarn supplying device 12 is caught by the second catching and guiding device 31 after the yarn Y is broken. In the first winder unit 3A, when the yarn Y is caught by the second catching and guiding device 31, the holding of the yarn Y by the holding device 21 is released. In the first winder unit 3A, when the holding of the yarn Y by the holding device 21 is released, the second catching and guiding device 31 is rotated from a catching position. Thus, the yarn Y located between the first gripping portion 21A and the second gripping portion 21B of the holding device 21 is taken out by the second catching and guiding device 31. Thus, in the first winder unit 3A, the yarn Y is removed from the holding device 21 after the tension of the yarn Y is measured.

In the first winder unit 3A, the elongation of the yarn Y can be calculated in the tension measurement of the yarn Y. In the first winder unit 3A, in the tension measurement of the yarn Y, the elongation of the yarn Y is calculated based on information monitored by the yarn monitoring device 24 when the yarn Y is being pulled by the yarn pulling device 25 in a state in which the yarn Y is caught by the holding device 21. Specifically, the section controller 10 calculates a displacement amount (a change in length of the yarn Y) of the yarn Y between the holding device 21 and the yarn pulling device 25 based on the information monitored by the yarn monitoring device 24, and calculates the elongation of the yarn Y between the holding device 21 and the yarn pulling device 25 based on the displacement amount. Thus, the elongation of the yarn Y can be calculated in the measurement of the tension of the yarn Y.

Alternatively, in the first winder unit 3A, the elongation of the yarn Y may be calculated based on time between a start and an end of the pulling of the yarn Y and a pulling speed of the yarn Y when the yarn Y is pulled by the yarn pulling device 25 in a state in which the yarn Y is held by the holding device 21. The pulling speed can be calculated from a rotation speed of the shaft portion 41A of the winding portion 41.

In the first winder unit 3A, the operation of the yarn joining device 23 is set based on the measurement result of the tension of the yarn Y. The section controller 10 sets the operation of the yarn joining device 23 based on the measurement result of the tension of the yarn joining portion, a preset threshold value or a reference value based on the measurement result of the tension of the yarn Y itself, and appearance data of the joint. For example, when the value of the measurement data of the tension of the yarn joining portion is lower than the threshold value, the section controller 10 sets various parameters of the yarn joining device 23 such that the value becomes greater than or equal to the threshold value set in experience (experiment) when the joint state changes in a direction in which the tension of the yarn joining portion increases. Thus, various parameters of the yarn joining device 23 can be automatically set. Therefore, the workload of the operator can be reduced.

As described above, in the automatic winder 1 according to the present embodiment, the first winder unit 3A includes the holding device 21 adapted to hold the yarn Y, the yarn pulling device 25 adapted to pull the yarn Y held by the holding device 21, and the tension measuring device 22 adapted to measure the tension of the yarn Y pulled by the yarn pulling device 25. Thus, the first winder unit 3A includes the holding device 21, the yarn pulling device 25, and the tension measuring device 22 as dedicated devices for measuring the tension of the yarn Y. Thus, in the first winder unit 3A, the yarn Y can be pulled by the pulling speed based on a predetermined tension test standard. Furthermore, the tension measuring device 22 can have performance capable of measuring the strength of the yarn Y conforming to the tension test standard. Therefore, in the first winder unit 3A, the measurement of the strength of the yarn Y conforming to the tension test standard can be realized.

In the first winder unit 3A according to the present embodiment, the yarn pulling device 25 is arranged to be movable in the forward-and-rearward direction with respect to the yarn path of the yarn Y. In this configuration, the yarn Y can be pulled at the position where the yarn Y deviates from the yarn path by moving the yarn pulling device 25. Therefore, the yarn Y can be avoided from being caught by the guidance, and the like at the time of measuring the tension. Thus, deterioration in the measurement accuracy of the tension of the yarn Y can be suppressed.

Although the embodiments of the present invention have been described above, the present invention is not necessarily limited to the above-described embodiments, and various modifications can be made without departing from the gist thereof.

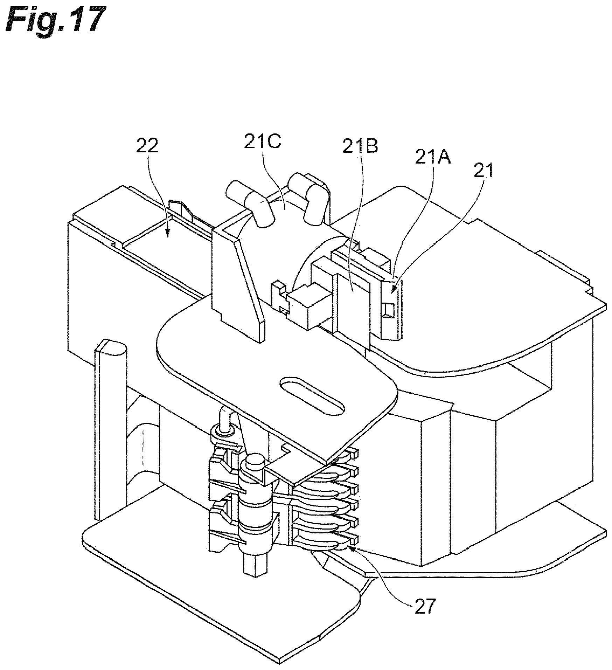

In addition to the embodiments described above, the first winder unit 3A may include the gate-type tension applying device 27. For example, as illustrated in FIG. 17, the gate-type tension applying device 27 may be provided integrally with the holding device 21 and the tension measuring device 22.

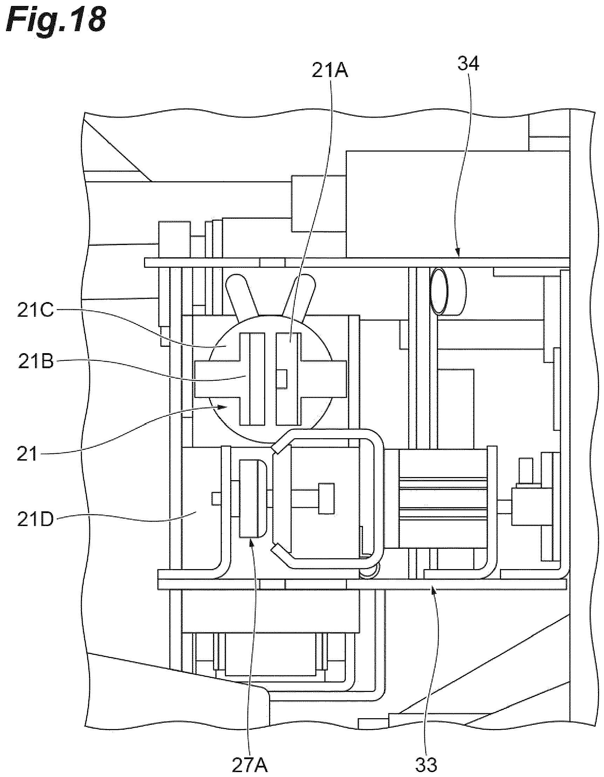

In place of the mode illustrated in FIG. 17, the first winder unit 3A may include a disc-type tension applying device 27A, as illustrated in FIG. 18. The disc-type tension applying device 27A may be provided integrally with the holding device 21 and the tension measuring device 22. The disc-type tension applying device 27A is in a closed state by the force of the coil spring, and sandwiches the yarn Y to apply tension. Furthermore, when the yarn Y is introduced to the disc-type tension applying device 27A, the disc becomes the opened state by a driving device such as an air cylinder.

As illustrated in FIG. 17 or 18, by integrally providing the tension applying devices 27 and 27A with the holding device 21 and the tension measuring device 22, the loosening of the yarn Y during the package winding can be prevented. For example, when the tension of the yarn joining portion joined by the yarn joining device 23 and the tension of the yarn Y itself are alternately measured a plurality of times, the tension test and the winding need to be repeatedly performed a plurality of times. In the present embodiment, the loosening of the yarn Y on the package surface can be prevented even at the time of this winding, so that disturbance does not occur on the package surface.

In the embodiment, a description has been given, as an example, as to a mode in which the holding device 21 holds the yarn Y by gripping the yarn Y using the first gripping portion 21A and the second gripping portion 21B. However, the holding section adapted to hold the yarn Y may have another configuration. For example, the yarn Y may be held by the second catching and guiding device 31. Specifically, the yarn Y may be held by catching the yarn Y in the second catching and guiding device 31 and closing a catching port with a lid. In this configuration, the yarn Y can be held by the existing device of the first winder unit 3A.

In the embodiment described above, the mode in which the yarn pulling device 25 includes the moving mechanism 44 has been described by way of example. However, the method of moving the winding portion 41 in the forward-and-rearward direction may be other means. For example, the winding portion 41 may be moved by the rotation of the driving section 42. Specifically, the winding portion 41 may be moved by driving the driving section 42 by a rack-and-pinion mechanism.

In addition to the embodiment described above, the first winder unit 3A may further include a yarn pulling device adapted to pull the yarn Y. The yarn pulling device is arranged between the unwinding assisting device 20 and the holding device 21. In this configuration, before the yarn Y is held by the holding device 21, the yarn Y is pulled by the yarn pulling device, and the yarn Y is held by the holding device 21 in a state in which a predetermined tension is applied to the yarn Y. For example, when the yarn Y is wound around the winding portion 41 of the yarn pulling device 25 in the case of a yarn having high elongation as in the case of spandex, the yarn Y may be wound around the winding portion 41 up to the yarn joining portion. Therefore, the amount of the yarn Y wound around the winding portion 41 can be reduced by pulling the yarn Y by a predetermined amount in the yarn pulling device arranged upstream of the holding device 21 and then holding the yarn Y by the holding device 21. Therefore, the tension of the yarn joining portion can be appropriately measured even with a yarn having high elongation as in the case of spandex.

In the embodiments described above, the mode of measuring the tension of the yarn Y by the tension measuring device 22, which is a load cell, has been described by way of example. However, the measuring section adapted to measure the tension of the yarn Y is not limited to the load cell. For example, the measuring section may be a device that is connected to the yarn pulling device 25 and measures the rotational torque of the winding portion 41.

In the embodiments, the measured measurement data (each calculated value such as the tension, the appearance data, the average value, and the like measured until the yarn Y is broken) is output to the section controller 10. The section controller 10 displays the measured tension of the yarn Y on the display section 26A of the display device 26. In addition, the measurement data can be further output from the section controller 10 to an external device (for example, a factory management terminal or a host server). An output method to the external device is not limited to a conventional wired connection, and an output by wireless communication or an output via a storage medium such as a flash memory can also be adopted.

In the embodiments, when the measured measurement data (each calculated value such as the tension, the appearance data, the average value, and the like measured until the yarn Y is broken) does not satisfy a predetermined condition, a mode for performing re-measurement may be provided. For example, when the measured tension measurement data when the yarn Y is broken is less than predetermined tension data, the tension measuring device 22 re-measures the tension until the yarn Y pulled by the yarn pulling device 25 is broken before the measurement result until the yarn Y is broken is output to the section controller 10. The re-measurement can be carried out both in the measurement of the tension of the yarn joining portion joined by the yarn joining device 23 and the measurement of the tension of the yarn Y itself. Further, the re-measured measurement data is recorded separately from the initial measurement data. As a result, it is possible to compare the data at the time of measurement failure and the data of re-measurement, and as such it is possible to verify a cause indicating that the first measurement data does not satisfy the predetermined condition.

## Claims

1. A yarn winding device (3A) comprising:
a yarn supplying section (12) configured to supply a yarn (Y);
a winding section (14) configured to wind the yarn (Y) supplied from the yarn supplying section (12) to form a package (P) ;
a holding section (21) configured to hold the yarn (Y) between the yarn supplying section (12) and the winding section (14) ;
a pulling section (25) configured to pull the yarn (Y) held by the holding section (21); and
a measuring section (22) configured to measure a tension of the yarn (Y) pulled by the pulling section (25),
wherein the measuring section (22) is connected to the holding section (21) or the pulling section (25).

2. The yarn winding device (3A) according to claim 1, wherein the measuring section (22) is a load cell and is connected to the holding section (21).

3. The yarn winding device (3A) according to claim 1, wherein
the pulling section (25) includes a winding portion (41) having a locking member (41D) for hooking the yarn (Y), the winding portion (41) being configured to wind the yarn (Y) hooked to the locking member (41D), and
the measuring section (22) is connected to the pulling section (25) and is configured to measure the rotational torque of the winding portion (41).

4. The yarn winding device (3A) according to claim 3, wherein
the pulling section (25) includes a driving section (21C) configured to rotationally drive the winding portion (41).

5. The yarn winding device (3A) according to claim 4, wherein
the driving section (21C) is configured to rotate, after measurement of the yarn (Y), the winding portion (41) in a direction opposite to a direction in which the winding portion (41) is rotated to wind the yarn (Y), and
the winding section (14) is configured to wind the yarn (Y) unwound from the winding portion (41) when the winding portion (41) is rotated in the direction opposite to the direction.

6. The yarn winding device (3A) according to any one of claims 1 to 5, wherein the pulling section (25) is arranged so as to be movable in a forward-and-rearward direction with respect to a yarn path of the yarn (Y).

7. The yarn winding device (3A) according to any one of claims 1 to 6, further comprising a display section configured to display information on the measurement of the yarn (Y) in the measuring section (22).

8. The yarn winding device (3A) according to any one of claims 1 to 7, further comprising:
an operating section (26B) configured to receive an input related to measurement of the tension of the yarn (Y); and
a control section configured to execute, based on the input received in the operating section (26B), an operation related to the measurement of the yarn (Y).

9. The yarn winding device (3A) according to any one of claims 1 to 8, further comprising:
a yarn joining section (23) configured to perform yarn joining of the yarn (Y) from the yarn supplying section (12) and the yarn from the winding section (14) between the holding section (21) and the pulling section (25);
a first catching section (30) configured to catch the yarn (Y) from the winding section (14) and to guide the yarn (Y) to the yarn joining section (23); and
a second catching section (31) configured to catch the yarn (Y) from the yarn supplying section (12) and to guide the yarn (Y) to the yarn joining section (23),
wherein the second catching section (31) is configured to catch, after the measurement of the yarn (Y), the yarn (Y) from the yarn supplying section (12) and to move the yarn (Y) above the holding section (21).

10. The yarn winding device (3A) according to claim 9, further comprising a setting section configured to change, based on a measurement result of the tension of the yarn (Y), a setting related to a yarn joining operation in the yarn joining section (23) .

11. The yarn winding device (3A) according to any one of claims 1 to 10, further comprising:
a yarn monitoring section (24) configured to monitor a state of the yarn (Y) travelling through the yarn path of the yarn (Y) between the holding section (21) and the pulling section (25), and to calculate a length of the yarn (Y) based on monitored information; and
a calculating section configured to calculate, when the yarn (Y) held by the holding section (21) is pulled by the pulling section (25), an elongation of the yarn (Y) based on the length of the yarn (Y) calculated by the yarn monitoring section (24).

12. The yarn winding device (3A) according to any one of claims 1 to 10, further comprising a calculating section configured to calculate, when the yarn (Y) held by the holding section (21) is pulled by the pulling section (25), an elongation of the yarn (Y) based on a time between a start and an end of pulling of the yarn (Y) by the pulling section (25) and a pulling speed of the yarn (Y).

13. An automatic winder (1) comprising:
a plurality of yarn winding units (3) configured to wind a yarn (Y) obtained by unwinding the yarn (Y) from a yarn supplying bobbin (SB) to form a package (P); and
a central control section (5) configured to control the plurality of yarn winding units (3),
wherein at least one of the plurality of yarn winding units (3) is the yarn winding device (3A) according to any one of claims 1 to 12.

14. The automatic winder according to claim 13, wherein
the central control section (5) is configured to be arranged at an end portion in a juxtaposition direction in which the plurality of yarn winding units (3) are arranged side by side, and
the yarn winding device (3A) is arranged at a position closest to the central control section (5) in the juxtaposition direction.
